# EUROPÄISCHE PATENTSCHRIFT

(11) **EP 1 803 438 B1**
(45) Veröffentlichungstag und Bekanntmachung des Hinweises auf die Patenterteilung: **02.09.2015**
(21) Anmeldenummer: 07001943.5
(22) Anmeldetag: 22.07.2000
(51) Int. Cl.: A61K 8/41, A61K 8/49, A61Q 5/10

(54) **Entwickler-Kuppler-Kombination**
Developer-coupler combination
Combinaison développeur-coupleur

(30) Priorität: 03.08.1999 DE 19936442
(43) Veröffentlichungstag der Anmeldung: 04.07.2007
(62) Teilanmeldung aus: 00949398.2
(73) Patentinhaber: Henkel AG & Co. KGaA, 40589 Düsseldorf (DE)
(72) Erfinder: Höffkes, Horst, 40595 Düsseldorf (DE); Rose, David, 40723 Hilden (DE)

(56) Entgegenhaltungen:
- EP-A2- 0 873 746
- EP-A2- 1 870 135
- DE-A1- 4 440 955
- DE-A1- 19 606 644
- DE-A1- 19 607 158
- DE-C1- 19 807 244
- FR-A- 2 751 218
- FR-A- 2 767 687
- FR-A- 2 769 211

## Beschreibung

Die vorliegende Erfindung betrifft Mittel zum Färben keratinischer Fasern, insbesondere menschlicher Haare, die Kombinationen spezieller Oxidationsfarbstoffvorprodukte enthalten, sowie deren Verwendung.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehrerer Kupplerkomponenten die eigentlichen Farbstoffe aus.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt. Als Kupplerkomponenten werden in der Regel m-Phenylendiaminderivate, Naphthole, Resorcin und Resorcinderivate, Pyrazolone und m-Aminophenole verwendet.

In DE 44 40 955 A1 werden oxidative Haarfärbemittel offenbart, welche eine Kombination aus dem Kuppler 2-Chlor-6-methyl-3-aminophenol und in Zweistellung und/oder in Dreistellung substituierten p-Aminophenolderivaten enthalten.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Es besteht daher ständig Bedarf an neuen, verbesserten Farbstoffkombinationen. Die durch die Farbstoffkombinationen erzielbaren Färbungen sollen beständig sein gegen Licht, Wärme, Schweiß, Reibung und den Einfluß chemischer Reduktionsmittel, z.B. Dauerwellenflüssigkeiten. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll. Daher bestand nach wie vor die Aufgabe, neue Entwickler-Kupplerkombinationen zu entwickeln, mit denen sich ausdrucksvolle, brillante Farbtöne, insbesondere im Rot/Rotbraun-Bereich, erzielen lassen und die auch in toxikologischer Hinsicht ein Fortschritt gegenüber dem Stand der Technik sind.

Es wurde nun überraschenderweise gefunden, daß bestimmte Farbstoffkombinationen die an Oxidationshaarfarben gestellten Aufgaben in einem hohen Maße erfüllen.

Ein erster Gegenstand der vorliegenden Erfindung sind daher Mittel zum Färben von Keratinfasern, insbesondere menschlichen Haaren, die in einem zum Färben geeigneten Medium mindestens ein p-Aminophenolderivat enthalten, und weiterhin
(a) 3-Amino-2-chlor-6-methylphenol und
(b) zur Nuancierung mindestens eine Kupplerkomponente ausgewählt aus 2,4-Diaminophenoxyethanol, 1-Methoxy-2-amino-4-(2'-hydroxyethylamino)-benzol, 1-Amino-3-bis-(2'-hydroxyethyl)-aminobenzol und 2,6-Bis-(2-hydroxyethylamino)-1-methylbenzol enthalten.

Unter Keratinfasern sind dabei Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Besonders bevorzugt im Sinne der vorliegenden Erfindung sind Oxidationsfärbemittel in einem wäßrigen Träger oder in Pulverform.

Beispiele für die als Substituenten in den erfindungsgemäßen Verbindungen genannten, C₁-bis C₄-Alkylgruppen sind die Gruppen Methyl, Ethyl, Propyl, Isopropyl und Butyl. Ethyl und Methyl sind bevorzugte Alkylgruppen. Eine erfindungsgemäß bevorzugte C₁- bis C₄-Alkoxygruppe, ist beispielsweise eine Methoxy- oder eine Ethoxygruppe. Diese Definition der Alkyl- und Alkoxygruppen ist auch anzuwenden, wenn diese Bestandteile eines komplexeren Substituenten sind. So sind bevorzugte Vertreter für eine C₁- bis C₄-Hydroxyalkylgruppe beispielsweise eine Hydroxymethyl-, eine 2-Hydroxyethyl-, eine 3-Hydroxypropyl- oder eine 4-Hydroxybutylgruppe. Beispiele für ein Halogenatom sind erfindungsgemäß ein F-, ein Cl- oder ein Br-Atom, ein Cl-Atom ist ganz besonders bevorzugt.

3-Amino-2-chlor-6-methylphenol wird eingesetzt, und es ist es erfindungswesentlich, daß eine weitere Kupplerkomponente anwesend ist. Durch diese Bedingung ist die Verbesserung der Echtheitseigenschaften der erzielbaren Färbungen gewährleistet. Insbesondere das Egalisiervermögen auf frisch nachgewachsenem sowie bereits vorgeschädigtem Haar wird auf diese Weise wesentlich verbessert. Geeignet sind die zur Erzielung einer Blauverschiebung der Nuancen geeigneten Kupplerkomponenten.

Erfindungsgemäß bevorzugte p-Aminophenol-Derivate sind ausgewählt aus Verbindungen der Formel (III) 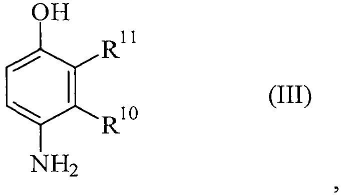 wobei
R¹⁰ und R¹¹ unabhängig voneinander stehen für Wasserstoff, eine C₁₋₄-Alkyl-Gruppe, eine C₁₋₄-Monohydroxyalkyl-Gruppe, eine C₂₋₄-Polyhydroxyalkyl-Gruppe, eine C₁₋₄-Alkoxy-C₁₋₄-Alkyl-Gruppe, eine C₁₋₄-Aminoalkyl-Gruppe, eine (C₁₋₄-Hydroxyalkyl)amino-Gruppe, eine ((C₁₋₄-Hydroxyalkyl)amino)-C₁₋₄-Alkyl-Gruppe, eine ((Di-C₁₋₄-Alkyl)-amino)-C₁₋₄-Alkyl-Gruppe, eine C₁₋₄-Hydroxyalkoxy-Gruppe, eine (2-Hydroxy-5-aminophenyl)-C₁₋₄-alkyl-Gruppe, eine Carboxy-Gruppe oder ein Halogenatom.

Die in dieser Definition verwendeten Begriffe sind analog zu den vorherigen Ausführungen definiert.

Obwohl prinzipiell alle p-Aminophenolderivate der Formel (III) im Sinne der Erfindung bevorzugt sind, sind p-Aminophenol und dessen Derivate Bis-(5-amino-2-hydroxyphenyl)-methan, 2-Aminomethyl-4-aminophenol, 2-(β-Hydroxyethylaminomethyl)-4-aminophenol, 3-Methyl-4-aminophenol, 5-Aminosalicylsäure, 2-Hydroxymethylamino-4-amino-phenol, 2-Hydroxymethyl-4-aminophenol, 4-Amino-2-((diethylamino)-methyl)-phenol, 4-Amino-3-fluorphenol, 4-Amino-2-chlorphenol und 4-Amino-2-(2-hydroxyethoxy)-phenol besonders geeignet. Ganz besonders geeignet sind Bis-(5-amino-2-hydroxyphenyl)-methan, 2-Aminomethyl-4-aminophenol, 4-Amino-2-((diethylamino)-methyl)-phenol, 2-(β-Hydroxyethylaminomethyl)-4-aminophenol, 3-Methyl-4-aminophenol, 4-Amino-2-chlorphenol und 5-Aminosalicylsäure.

Zur Erzielung natürlicher Nuancen ist es häufig notwendig, auch Farbstoffkombinationen einzusetzen, die der Färbung eine gewissen Blau- bzw. Anthrazitanteil geben. Hierzu können prinzipiell sowohl geeignete Entwickler- als auch geeignete Kupplerkomponenten eingesetzt werden. Zu diesem Zweck bevorzugt eingesetzte Entwicklerkomponenten sind p-Phenylendiamin, p-Toluylendiamin, N,N-Bis-(2-hydroxyethyl)-p-phenylendiamin, 1-(2'-Hydroxyethyl)-2,5-diaminobenzol, Bis-(4-aminophenyl)-amin, 1,10-Bis-(2,5-diaminophenyl)-1,4,7,10-tetraoxadecan, 2,6-Dichlor-4-aminophenol, 2,5-Diaminopyridin und 2-Dimethylamino-5-aminopyridin. Als Kupplerkomponenten werden 2,4-Diaminophenoxyethanol, 1-Methoxy-2-amino-4-(2'-hydroxyethylamino)-benzol, 1-Amino-3-bis-(2'-hydroxyethyl)-aminobenzol und 2,6-Bis-(2-hydroxyethylamino)-1-methylbenzol, zur Erzielung der genannten Nuancen eingesetzt.

Neben der erfindungsgemäßen Kombination von Oxidationsfarbstoffvorprodukten können die Mittel zur weiteren Nuancierung weitere Oxidationsfarbstoffvorprodukte vom Kuppler- und Entwicklertyp enthalten.

Erfindungsgemäß bevorzugte weitere Entwicklerkomponenten sind o-Aminophenol, 2-(2,5-Diaminophenoxy)-ethanol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 1,4-Bis-(4-aminophenyl)-diazacycloheptan, 1,3-Bis-(N(2-hydroxyethyl)-N-(4-aminophenylami-no))-2-propanol, 1,10-Bis-(2,5-diaminophenyl)-1,4,7,10-tetraoxadecan sowie 4,5-Diamino-pyrazol-Derivate nach EP 0 740 741 bzw. WO 94/08970 wie z.B. 4,5-Diamino-1-(2'-hydroxyethyl)-pyrazol.

Besonders bevorzugte weitere Entwicklerkomponenten sind 2,4,5,6-Tetraaminopyrimidin, 2-Hydroxy-4,5,6-triaminopyrimidin, 4-Hydroxy-2,5,6-triaminopyrimidin, 4,5-Diamino-1-(2-hydroxyethyl)-pyrazol und o-Aminophenol.

Erfindungsgemäß bevorzugte weitere Kupplerkomponenten sind
- m-Aminophenol und dessen Derivate wie beispielsweise 5-Amino-2-methylphenol, 5-(3-Hydroxypropylamino)-2-methylphenol, 2-Hydroxy-4-aminophenoxyethanol, 2,6-Dimethyl-3-aminophenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol und 3-(Ethylamino)-4-methylphenol,
- o-Aminophenol und dessen Derivate,
- m-Diaminobenzol-Derivate,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol, Pyridinderivate wie beispielsweise 2,6-Dihydroxypyridin, 2-Amino-3-hydroxypyridin, 2,6-Dihydroxy-3,4-dimethylpyridin und 2,6-Dihydroxy-4-methylpyridin,
- Naphthalinderivate wie beispielsweise 2-Methyl-1-naphthol, 2-Hydroxymethyl-1-naphthol, 2-Hydroxyethyl-1-naphthol, 1,6-Dihydroxynaphthalin, 1,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol sowie
- Methylendioxybenzolderivate.

Erfindungsgemäß bevorzugte weitere Kupplerkomponenten sind 1,7-Dihydroxynaphthalin, 2-Amino-3-hydroxypyridin, 5-Amino-2-methylphenol, Resorcin, 4-Chlorresorcin, 2-Methylresorcin, 5-Methylresorcin, 2,5-Dimethylresorcin, 2,6-Dihydroxy-3,4-diaminopyridin, 6-Methyl-1,2,3,4-tetrahydrochinoxalin, m-Aminophenol, o-Aminophenol und 2-Chlorresorcin.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1:0,5 bis 1:3, insbesondere 1:1 bis 1:2, enthalten sein können.

In einer bevorzugten Ausführungsform enthalten die erfindungsgemäßen Haarfärbemittel zur weiteren Modifizierung der Farbnuancen neben den Oxidationsfarbstoffvorprodukten zusätzlich übliche direktziehende Farbstoffe. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Zur Herstellung der erfindungsgemäßen Färbemittel können die Oxidationsfarbstoffvorprodukte in einen geeigneten wasserhaltigen Träger eingearbeitet werden. Solche Träger sind zum Zwecke der Haarfärbung z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammo-niumchloride und Trialkylmethylammoniumchloride, z. B. Cetyltrimethylammo-niumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine, wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind quaternäre Esterverbindungen, sogenannte "Esterquats", wie die unter dem Warenzeichen Stepantex^{®} vertriebenen Methyl-hydroxyalkyldialkoyloxyalkyl-ammoniummethosulfate sowie die unter dem Warenzeichen Dehyquart^{®} vertriebenen Produkte wie Dehyquart^{®} AU-46.

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen; können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die erfindungsgemäßen Haarbehandlungsmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate;
- polymere Dimethyldiallylammoniumsalze und deren Copolymere mit Acrylsäure sowie Estern und Amiden von Acrylsäure und Methacrylsäure. Die unter den Bezeichnungen Merquat^{®} 100 (Poly(dimethyldiallylammoniumchlorid)), Merquat^{®}550 (Dimethyldiallylammoniumchlorid-Acrylamid-Copolymer) und Merquat^{®} 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Methoimidazoliniumchlorid-Copolymere, wie sie unter der Bezeichnung Luviquat^{®} angeboten werden;
- quaternierter Polyvinylalkohol
   sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen. Dabei können die Enzyme (Enzymklasse 1: Oxidoreduktasen) Elektronen aus geeigneten Entwicklerkomponenten (Reduktionsmittel) auf Luftsauerstoff übertragen. Bevorzugt sind Oxidasen wie Tyrosinase und Laccase aber auch Glucoseoxidase, Uricase oder Pyruvatoxidase. Weiterhin können die Enzyme zur Verstärkung der Wirkung geringer Mengen vorhandener Oxidationsmittel dienen. Ein Beispiel für ein derartiges enzymatisches Verfahren stellt das Vorgehen dar, die Wirkung geringer Mengen (z.B. 1% und weniger, bezogen auf das gesamte Mittel) Wasserstoffperoxid durch Peroxidasen zu verstärken.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Ein zweiter Gegenstand dieser Erfindung ist die Verwendung der vorgenannten Mittel zum Färben keratinischer Fasern.

## Patentansprüche

1. Oxidationsfärbemittel zum Färben von Keratinfasern, insbesondere menschlichen Haaren, enthaltend in einem zum Färben geeigneten Medium mindestens ein p-Aminophenolderivat und 3-Amino-2-chlor-6-methylphenol, **dadurch gekennzeichnet, dass** das Mittel zur Nuancierung mindestens eine Kupplerkomponente ausgewählt aus 2,4-Diaminophenoxyethanol, 1-Methoxy-2-amino-4-(2'-hydroxyethylamino)-benzol, 1-Amino-3-bis-(2'-hydroxyethyl)-aminobenzol und 2,6-Bis-(2-hydroxyethylamino)-1-methylbenzol enthält.

2. Mittel nach Anspruch 1, **dadurch gekennzeichnet, dass** das p-Aminophenol-Derivat ausgewählt ist aus Verbindungen der Formel (III) 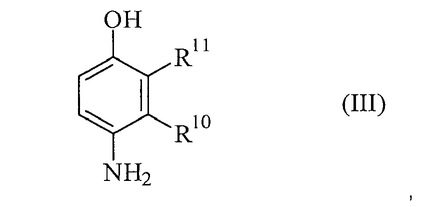 wobei
R¹⁰ und R¹¹ unabhängig voneinander stehen für Wasserstoff, eine C₁₋₄-Alkyl-Gruppe, eine C₁₋₄-Monohydroxyalkyl-Gruppe, eine C₂₋₄-Polyhydroxyalkyl-Gruppe, eine C₁₋₄-Alkoxy-C₁₋₄-Alkyl-Gruppe, eine C₁₋₄-Aminoalkyl-Gruppe, eine (C₁₋₄-Hydroxyalkyl)amino-Gruppe, eine ((C₁₋₄-Hydroxyalkyl)-amino)-C₁₋₄-Alkyl-Gruppe, eine ((Di-C₁₋₄-Alkyl)-amino)-C₁₋₄-Alkyl-Gruppe, eine C₁₋₄-Hydroxyalkoxy-Gruppe, eine (2-Hydroxy-5-aminophenyl)-C₁₋₄-alkyl-Gruppe, eine Carboxy-Gruppe oder ein Halogenatom.

3. Mittel nach einem der Ansprüche 1 oder 2, **dadurch gekennzeichnet, dass** die Verbindung der Formel (III) ausgewählt ist aus Bis-(5-amino-2-hydroxyphenyl)-methan, 2-Aminomethyl-4-aminophenol, 4-Amino-2-((diethylamino)-methyl)-phenol, 2-(β-Hydroxyethylaminomethyl)-4-aminophenol, 3-Methyl-4-aminophenol und 5-Aminosalicylsäure.

4. Mittel nach einem der Ansprüche 1 bis 3, **dadurch gekennzeichnet, dass** es zur Nuancierung mindestens eine Entwicklerkomponente, ausgewählt aus der Gruppe, die aus p-Phenylendiamin, p-Toluylendiamin, N,N-Bis-(2-hydroxy-ethyl)-p-phenylendiamin, 1-(2'-Hydroxyethyl)-2,5-diaminobenzol Bis-(4-aminophenyl)-amin, 1,10-Bis-(2,5-diaminophenyl)-1,4,7,10-tetraoxadecan, 2,6-Dichlor-4-aminophenol, 2,5-Diaminopyridin und 2-Dimethylamino-5-aminopyridin gebildet wird, enthält.

5. Mittel nach einem der Ansprüche 1 bis 4, **dadurch gekennzeichnet, dass** Entwicklerkomponenten in einer Menge von 0,005 bis 20 Gew.-%, und Kupplerkomponenten in einer Menge von 0,005 bis 20 Gew.-%, jeweils bezogen auf das gesamte Oxidationsfärbemittel, enthalten sind.

6. Mittel nach einem der Ansprüche 1 bis 6, **dadurch gekennzeichnet, dass** mindestens ein direktziehender Farbstoff enthalten ist.

7. Verwendung von Mitteln nach einem der Ansprüche 1 bis 6 zum Färben keratinischer Fasern.

## Claims

1. Oxidation dye for dyeing keratin fibers, in particular human hair, which in a medium suitable for dyeing contains at least one p-aminophenol derivative and 3-amino-2-chloro-6-methylphenol, **characterized in that**, for shading, the agent contains at least one coupler component selected from 2,4-diaminophenoxyethanol, 1-methoxy-2-amino-4-(2'-hydroxyethylamino)benzene, 1-amino-3-bis-(2'-hydroxyethyl)aminobenzene, and 2,6-bis-(2-hydroxyethylamino)-1-methylbenzene.

2. Agent according to Claim 1, **characterized in that** the p-aminophenol derivative is selected from compounds of formula (III) 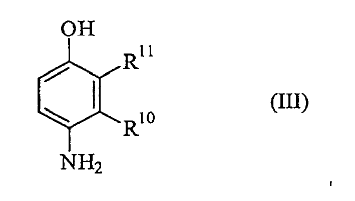 where
R¹⁰ and R¹¹ independently stand for hydrogen, a C₁-₄ alkyl group, a C₁₋₄ monohydroxyalkyl group, a C₂₋₄ polyhydroxyalkyl group, a C₁₋₄ alkoxy-C₁₋₄ alkyl group, a C₁₋₄ aminoalkyl group, a (C₁₋₄-hydroxyalkyl)amino group, a ((C₁₋₄-hydroxyalkyl)amino)-C₁₋₄ alkyl group, a ((di-C₁₋₄ alkyl)amino)-C₁₋₄ alkyl group, a C₁₋₄-hydroxyalkoxy group, a (2-hydroxy-5-aminophenyl)-C₁₋₄ alkyl group, a carboxy group, or a halogen atom.

3. Agent according to one of Claims 1 or 2, **characterized in that** the compound of formula (III) is selected from bis-(5-amino-2-hydroxyphenyl)methane, 2-aminomethyl-4-aminophenol, 4-amino-2-((diethylamino)methyl)phenol, 2-(β-hydroxyethylaminomethyl)-4-aminophenol, 3-methyl-4-aminophenol, and 5-aminosalicylic acid.

4. Agent according to one of Claims 1 to 3, **characterized in that**, for shading, it contains at least one developer component selected from the group comprising *p*-phenylenediamine, *p*-toluylenediamine, *N,N*-bis-(2-hydroxyethyl)-*p-*phenylenediamine, 1-(2'-hydroxyethyl)-2,5-diaminobenzene, bis-(4-aminophenyl)amine, 1,10-bis-(2,5-diaminophenyl)-1,4,7,10-tetraoxadecane, 2,6-dichloro-4-aminophenol, 2,5-diaminopyridine, and 2-dimethylamino-5-aminopyridine.

5. Agent according to one of Claims 1 to 4, **characterized in that** it contains developer components in a quantity of 0.005 to 20% by weight and coupler components in a quantity of 0.005 to 20% by weight, in each case based on the overall oxidation dye.

6. Agent according to one of Claims 1 to 6, **characterized in that** it contains at least one direct dye.

7. Use of agents according to one of Claims 1 to 6 for dyeing keratinic fibers.

## Revendications

1. Colorant d'oxydation servant à la coloration de fibres de kératine, et plus particulièrement de cheveux humains, comprenant dans un milieu approprié à la coloration au moins un dérivé du p-aminophénol et du 3-amino-2-chloro-6-méthylphénol, **caractérisé en ce que** l'agent de nuançage contient au moins un composant coupleur choisi parmi le 2,4-diaminophénoxyéthanol, le 1-méthoxy-2-amino-4-(2'-hydroxyéthylamino)-benzène, le 1-amino-3-bis-(2'-hydroxyéthyl)-aminobenzène et le 2,6-bis-(2'-hydroxyéthylamino)-1-méthylbenzène.

2. Colorant selon la revendication 1, **caractérisé en ce que** le dérivé du p-aminophénol est choisi parmi les composés de la formule (III) 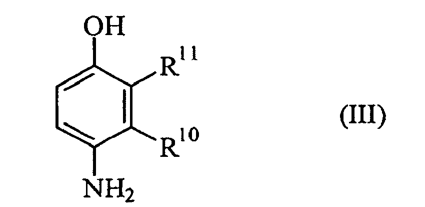 dans laquelle
R¹⁰ et R¹¹ sont indépendamment l'un de l'autre un atome d'hydrogène, un groupe alkyle en C₁ à C₄, un groupe monohydroxyalkyle en C₁ à C₄, un groupe polyhydroxyalkyle en C₂ à C₄, un groupe alcoxy en C₁ à C₄-alkyle en C₁ à C₄, un groupe aminoalkyle en C₁ à C₄, un groupe (hydroxyalkyle en C₁ à C₄)-amino, un groupe (hydroxyalkyle en C₁ à C₄)-amino)-alkyle en C₁ à C₄, un groupe ((di-alkyle en C₁ à C₄)-amino)-alkyle en C₁ à C₄, un groupe hydroxyalcoxy en C₁ à C₄, une (2-hydroxy-5-aminophényle)-alkyle en C₁ à C₄, un groupe carboxy ou un atome d'halogène.

3. Colorant selon l'une des revendications 1 ou 2, **caractérisé en ce que** le composé de la formule (III) est choisi parmi le bis-(5-amino-2-hydroxyphényl)-méthane, le 2-aminométhyl-4-aminophénol, le 4-amino-2-((diéthylamino)-méthyl)-phénol, le 2-(β-hydroxyéthyl)-4-aminophénol, le 3-méthyl-4-aminophénol et l'acide 5-aminosalicylique.

4. Colorant selon l'une des revendications 1 à 3, **caractérisé en ce qu'**il contient pour le nuançage au moins un composant révélateur, choisi dans le groupe comportant la p-phénylènediamine, la p-toluènediamine, la N,N-bis-(2-hydroxy-éthyl)-p-phénylènediamine, le 1-(2-hydroxyéthyl)-2,5-diaminobenzène, la bis-(4-aminophényl)-amine, le 1,10-bis-(2,5-diaminophényl)-1,4,7,10-tétraoxadécane, le 2,6-dichloro-4-aminophénol, la 2,5-diaminopyridine et la 2-diméthylamino-5-aminopyridine.

5. Colorant selon l'une des revendications 1 à 4, **caractérisé en ce que** les composants révélateurs sont contenus dans une quantité de 0,005 à 20% en poids et les composants de couplage sont contenus dans une quantité de 0,005 à 20% en poids, rapportées à chaque fois à tout le colorant d'oxydation.

6. Colorant selon l'une des revendications 1 à 6, **caractérisé en ce qu'**il contient au moins un colorant direct.

7. Utilisation de colorants selon l'une des revendications 1 à 6, pour la coloration de fibres de kératine.
